# EUROPÄISCHE PATENTSCHRIFT

(11) **EP 2 575 731 B1**
(45) Veröffentlichungstag und Bekanntmachung des Hinweises auf die Patenterteilung: **26.11.2014**
(21) Anmeldenummer: 11723255.3
(22) Anmeldetag: 18.05.2011
(51) Int. Cl.: A61N 1/36, A61H 39/00, A61N 1/04, A61N 1/05

(54) **ELEKTRODENANORDNUNG**
ELECTRODE ARRANGEMENT
ENSEMBLE D'ÉLECTRODES

(30) Priorität: 04.11.2010 DE 202010014951 U; 28.05.2010 DE 102010021877
(43) Veröffentlichungstag der Anmeldung: 10.04.2013
(73) Patentinhaber: Cerbomed GmbH, 91052 Erlangen (DE)
(72) Erfinder: ELLRICH, Jens, 91094 Langensendelbach (DE)
(74) Vertreter: Gosdin, Michael
(86) Internationale Anmeldenummer: PCT/EP2011/002453
(87) Internationale Veröffentlichungsnummer: WO 2011/147546

(56) Entgegenhaltungen:
- WO-A1-92/08516
- US-A- 4 267 838
- US-A- 4 966 164
- US-A1- 2006 064 139
- US-A1- 2008 249 594

## Beschreibung

Die Erfindung betrifft eine Elektrodenanordnung zur Anbringung am und/oder im Ohr eines Menschen, wobei die Elektrodenanordnung ausgebildet ist, um auf die Oberfläche des Ohres einen transkutanen elektrischen Stimulationsreiz auszuüben, wobei die Elektrodenanordnung mindestens eine Stimulationselektrode und mindestens eine Referenzelektrode aufweist, wobei die mindestens eine Stimulationselektrode eine gebogene Struktur aufweist und die Oberfläche des Ohres über eine erste Kontaktfläche kontaktiert, wobei die mindestens eine Referenzelektrode eine ovale oder nierenförmige Struktur aufweist und die Oberfläche des Ohres über eine zweite Kontaktfläche kontaktiert, wobei die zweite Kontaktfläche mindestens 3 mal so groß ist wie die erste Kontaktfläche.

Es ist generell bekannt, durch invasive und non-invasive Reizung der Nerven Einfluss auf deren neurophysiologische und neuroelektrische Qualität und damit auf die Funktion der stimulierten Nerven zu nehmen. Hierdurch können verschiedene Krankheitszustände behandelt werden. Es existieren zahlreiche Vorrichtungen sowohl zur invasiven als auch zu non-invasiven Stimulation.

Die vorliegende Erfindung stellt auf die Methode der transkutanen elektrischen Nervenstimulation ab. Bei diesem Verfahren werden Impulsströme verschiedener Stromformen, Amplituden, Impulsdauern und Frequenzen durch die Haut hindurch an verschiedenen Nerven appliziert und verändern deren Statuspararneter in vorteilhafter Weise.

Eine Elektrodenanordnung der eingangs genannten Art ist aus der DE 10 2005 003 735 B4 bekannt. Hier ist eine Vorrichtung zur transkutanen Stimulation des Vagusnervs des menschlichen Körpers beschrieben, die einen zur Einführung in den Gehörgang vorgesehenen bügelförmigen Fortsatz aufweist, der an seinem in den Gehörgang einzuführenden Ende einen Elektrodenkopf aufweist. Hier sind in Richtung der Achse des Gehörgangs beabstandet zwei punktförmige Elektroden angeordnet. Mit dieser vorbekannten Lösung kann bereits eine effektive transkutane Stimulation insbesondere des Bereichs im Gehörgang erfolgen, wo der Vagusnerv verläuft. Allerdings sind die stimulierbaren Areale begrenzt.

Eine gattungsgemäße Lösung zeigt auch die US 4 267 838. Ähnliche und andere Stimulationsvorrichtungen sind in der WO 92/08516, in der US 4 966 164 und in der US 2006/0064139 A1 beschrieben.

Eine andere Elektrodenanordnung, die in der Pinna des Ohrs untergebracht werden kann, geht aus der DE 10 2006 023 824 A1 hervor. Hier sind Elektroden am Ende federnd ausgebildeter Halteelemente vorgesehen, mittels derer die Elektrodenanordnung im Ohr festgespannt werden kann.

Aus der US 2003/0195588 A1 ist ein nach Art eines Gehörgang-Stöpsels ausgebildeter Elektrodenkopf bekannt, der Elektroden in Form geschlossener Ringe aufweist. Auch hiermit ist eine transkutane Stimulation möglich. Allerdings ergeben sich aufgrund der relativ starren Struktur des Elektrodenkopfes Einschränkungen, wenn es um die Anpassungsfähigkeit der Elektroden an die innere Oberfläche des Gehörgangs geht.

Eine grundsätzlich andere Nervenstimulation ist in der US 3 449 768 und in der US 5 649 970 beschrieben. Hier kommen Stimulationselektroden zum Einsatz, die im Bereich des Ohrs in den Patienten implantiert werden.

Bei den vorbekannten Lösungen - soweit die hier interessierende Technologie der transkutanen Nervenstimulation in Abgrenzung zur Stimulation mit implantierten Elektroden betroffen ist - haben die Elektroden, d. h. die Stimulationselektrode und die Referenzelektrode, eine weitgehend gleiche Form und Größe. Dabei kommen beispielsweise zwei Metallelektroden mit kugelförmiger Oberfläche zum Einsatz, die in einem definierten Abstand angeordnet sind. Bekannt ist es auch, dass als Elektroden ringförmige metallische Elemente eingesetzt werden, die ebenfalls in einem definierten Abstand zueinander angeordnet sind.

Es hat sich herausgestellt, dass diese Ausgestaltung der Elektroden nicht immer zu einem optimalen Behandlungsergebnis führt. Vielmehr scheint eine andere Konzeption der Elektrodenform und -größe zu einem besserem Stimulationsergebnis zu führen. In diesem Zusammenhang stellt es ein besonderes Augenmerk bzw. Problem dar, dass es aufgrund der Behaarung der Hautoberfläche und aufgrund einer gerade im Ohrbereich vorhandenen Fettschicht auf der Hautoberfläche nicht unproblematisch ist, reproduzierbar eine definierte Stärke der transkutanen Nervenstimulation zu erreichen.

Der Erfindung liegt daher die **Aufgabe** zugrunde, eine Elektrodenanordnung der gattungsgemäßen Art so fortzubilden, dass dem genannten Nachteil Rechnung getragen werden kann. Demgemäß soll eine Elektrodenanordnung geschaffen werden, die so ausgestaltet ist, dass ein verbessertes Behandlungsergebnis bei der Applikation eines transkutanen elektrischen Stimulationsreizes erzielt werden kann. Dabei wird insbesondere eine möglichst hohe Unempfindlichkeit der Stimulation hinsichtlich der Behaarung der Hautoberfläche und einer gegebenenfalls sich auf dieser befindlichen Fettschicht angestrebt.

Die **Lösung** dieser Aufgabe durch die Erfindung ist dadurch gekennzeichnet, dass mindestens eine der Elektroden die Oberfläche des Ohres über eine Kontaktfläche kontaktiert, wobei die Elektrode so ausgebildet ist, dass sie mindestens 50 % der Oberfläche der Cymba conchae des Ohrs abdeckt. Bevorzugt ist die zweite Kontaktfläche mindestens 5 mal so groß ist wie die erste Kontaktfläche.

Die mindestens eine Stimulationselektrode fungiert dabei bevorzugt als Kathode und die mindestens eine Referenzelektrode als Anode in dem elektrischen Stromkreis, der während der transkutanen Stimulation geschlossen ist. Die Kathode ist bekanntlich die Elektrode, an der Reduktionsreaktionen ablaufen und die Elektronen abgibt. Die Kathode kann negative Polarität haben, wie bei einem elektrischen Verbraucher, oder positive Polarität, wie bei einem elektrischen Erzeuger beispielsweise einer Spannungsquelle. Die Kathode ist die Gegenelektrode zur Anode. Kationen wandern zur Kathode und Anionen zur Anode.

Die Stimulationselektrode kann mit ihrer gebogenen Struktur der Umfangsform des Tragus des Ohres angepasst sein. Dies kann sowohl die Außenseite des Tragus als auch dessen Innenseite sein. Die Stimulationselektrode hat dabei bevorzugt eine sichelförmige Struktur.

Die Referenzelektrode kann mit ihrer ovalen oder nierenförmigen Struktur der Form eines weitgehend ebenen Bereichs der Oberfläche der Pinna des Ohres angepasst sein.

Die mindestens eine Stimulationselektrode und die mindestens eine Referenzelektrode sind bei bestimmungsgemäßem Gebrauch zueinander vorzugsweise in einem Abstand angeordnet, der zwischen 5 mm und 50 mm beträgt.

Die Stimulationselektrode und die Referenzelektrode können aus mindestens einem metallischen Körper bestehen. Der metallische Körper kann an oder in einem Trägerkörper angeordnet sein, der aus elastischem Material besteht. Das elastische Material ist dabei bevorzugt ein Kunststoff, insbesondere ein biokompatibles Elastomermaterial, besonders bevorzugt Silikon oder ein Material, das Silikon aufweist.

Die Elektrodenanordnung kann zumindest teilweise aus einem leitfähigen Kunststoff bestehen. Sie kann auch aus einem Kunststoff bestehen, der zumindest abschnittsweise mit einer leitfähigen Oberfläche versehen ist. Die Leitfähigkeit des Kunststoffs bzw. der Kunststoffoberfläche kann zur Verwirklichung der Elektroden genutzt werden.

Die Elektrode oder der Elektrodenträger sind dabei bevorzugt so ausgebildet, dass sie bzw. er mindestens 80 % der Oberfläche der Cymba conchae des Ohrs abdeckt.

Des weiteren kann vorgesehen sein, dass eine weitere Elektrode oder ein diese tragender Elektrodenträger die Oberfläche des Ohres über eine weitere Kontaktfläche kontaktiert, wobei die Elektrode oder der Elektrodenträger so ausgebildet ist, dass sie bzw. er einen Teil der Antihelix des Ohrs abdeckt. Die Erfindung sieht also unterschiedlich große und bevorzugt auch unterschiedlich geformte und unterschiedlich gepolte Elektroden vor. Dabei ist bevorzugt vorgesehen, dass die als Kathode fungierende Stimulationselektrode direkt an der Stelle der größten subkutanen Konzentration des aurikulären Vagusnervs platziert wird, damit durch den abgegebene negativen Ladungsüberschuss die darunterliegenden Nerven depolarisiert werden. Durch die vergleichsweise zu den Referenzelektroden kleinen Elektrodenflächen ergibt sich eine entsprechend höhere Stromdichte, wodurch die Wahrscheinlichkeit steigt, dass die oben genannten Vagusäste depolarisiert werden. Die als Anode fungierende Referenzelektrode wird dann im Abstand zur Stimulationselektrode auf der benachbarten Haut angeordnet. Dabei ist allerdings ein zu großer Abstand zu vermeiden, damit nicht unnötig viel Körpergewebe bei der transkutanen Stimulation vom Strom durchflossen wird und keine zu hohe Stromstärke benötigt wird.

Vorteilhaft ist weiterhin, dass durch die vorgeschlagene Ausgestaltung der Elektrodenanordnung eine sehr einfache Anwendung durch den Benutzer möglich ist, da vorzugsweise kein Einführen eines Abschnitts der Elektrodenanordnung in den Gehörgang erforderlich ist.

Durch die vorgeschlagene Wahl der Flächenverhältnisse zwischen den Elektroden wird insbesondere erreicht, dass in vorteilhafter Weise eine relativ hohe Unempfindlichkeit der transkutanen Nervenstimulation gegen Behaarung und Befettung der Hautoberfläche gegeben ist.

In der Zeichnung sind Ausführungsbeispiele der Erfindung dargestellt. Es zeigen:
- Fig. 1: eine Ohrmuschel (Pinna) eines Menschen,
- Fig. 2: die Ohrmuschel mit einer Elektrodenanordnung, die auf definierte Bereiche des Ohrs aufgesetzt ist, um eine transkutane Stimulation vorzunehmen,
- Fig. 3: die Elektroden, die bei der Elektrodenanordnung gemäß Fig. 2 eingesetzt werden,
- Fig. 4: die Ohrmuschel mit einer Elektrodenanordnung, die speziell auf den Bereich der Cymba conchae aufgesetzt ist, um eine transkutane Stimulation vorzunehmen,
- Fig. 4a: eine zu Fig. 4 alternative Ausgestaltung der Elektrodenanordnung und
- Fig. 4b: eine weitere zu Fig. 4 alternative Ausgestaltung der Elektrodenanordnung.

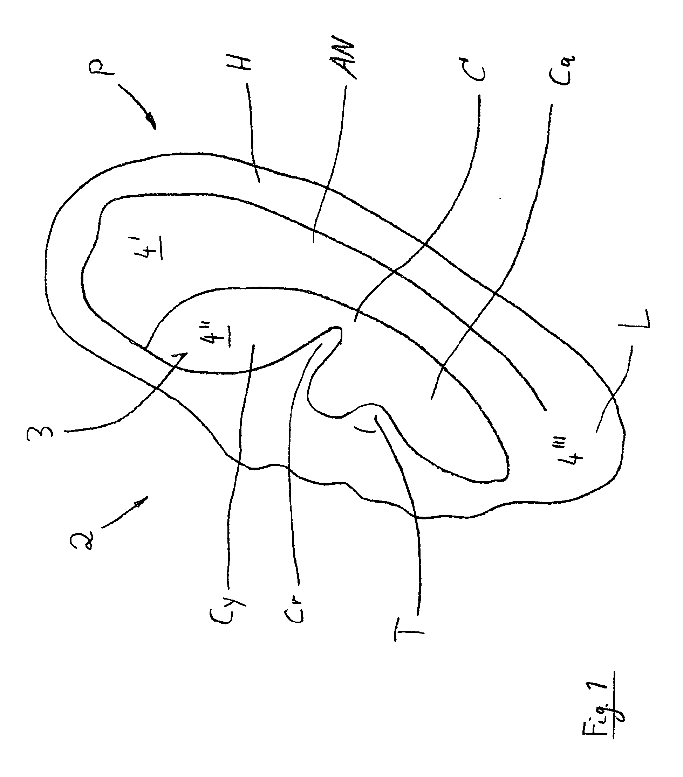

In Fig. 1 ist ein (Außen)Ohr 2 eines Menschen skizziert, dessen Form durch die Pinna (Ohrmuschel) P definiert ist. Die Pinna P umfasst in bekannter Weise die Helix H und Antihelix AN; zentral ist die Concha C angeordnet, die seitlich vom Tragus T begrenzt wird. Im unteren Bereich befindet sich die Lobule L. Die Concha C unterteilt sich in einen oberen und einen unteren Bereich; beide Bereiche werden durch die Crus helicis Cr voneinander abgetrennt. Der obere Teil der Concha C ist die Cymba conchae Cy, der untere Teil ist das Cavum conchae Ca.

Im Rahmen der vorliegenden Erfindung ist vorgesehen, dass spezielle Bereiche des Ohrs 2 einer transkutanen Stimulation ausgesetzt werden. Zur Anbringung einer als Kathode wirkenden Stimulationselektrode ist eine Oberfläche 3 des Ohres 2 vorgesehen, wobei es sich hier um die Innenseite des Tragus T handelt. Zur Anordnung einer als Anode wirkenden Referenzelektrode können alternativ oder additiv mehrere Zonen vorgesehen werden, wobei eine Oberfläche 4' im oberen Bereich der Antihelix AN, eine Oberfläche 4" im oberen Bereich der Concha C und/oder eine Oberfläche 4'" im Bereich der Lobule L bevorzugt ist.

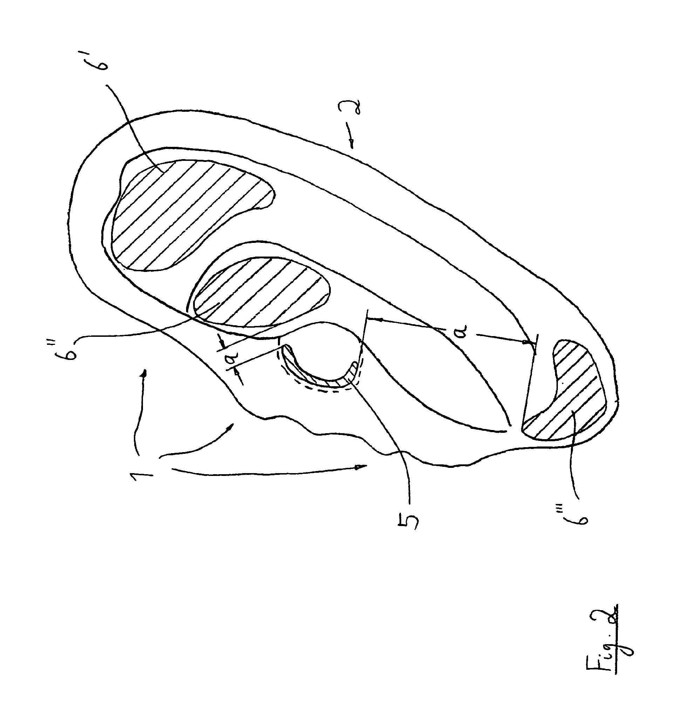

In Fig. 2 ist dargestellt, wie eine Elektrodenanordnung 1 am bzw. im Ohr 2 platziert wird, um auf die Oberflächen 3, 4 eine transkutane Stimulation auszuüben.

Die Elektrodenanordnung 1 ist hier nur betreffend ihre Elektroden 5 und 6 skizziert. Weitere Elemente (evtl. Gehäuse und elektrische Anschlüsse) sind nicht dargestellt. Die nötigen Mittel sind im Stand der Technik hinlänglich bekannt, so dass sie hier nicht weiter beschrieben werden müssen. Exemplarisch wird auf die DE 10 2005 003 753 B4 der Anmelderin verwiesen und hierauf ausdrücklich Bezug genommen.

Mittels der Elektroden 5, 6 kann auf die Oberflächen 3 und 4 des Ohres (s. Fig. 1) und insbesondere dort, wo der Vagusnerv verläuft, eine transkutane elektrische Nervenstimulation vorgenommen werden. Zwischen der Stimulationselektrode 5 und der (mindestens einen) Referenzelektrode 6 wird hiefür ein elektrisches Potential erzeugt.

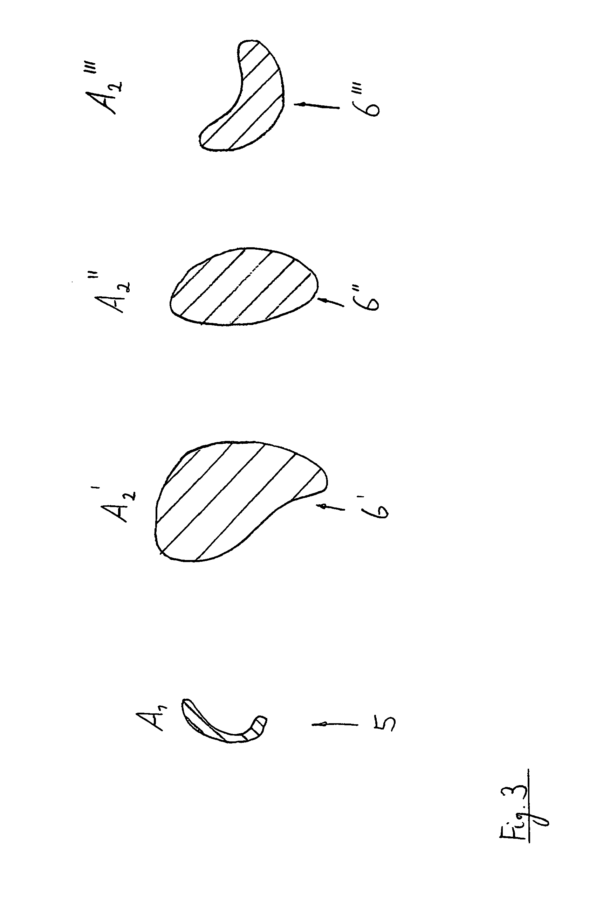

Wie in Fig. 2 und Fig. 3 erkannt werden kann, weist die Stimulationselektrode 5 im Ausführungsbeispiel eine gebogene, sichelförmige Gestalt auf. Die Fläche der Stimulationselektrode 5, mit der diese die Oberfläche 3 des Ohres 2, im vorliegenden Falle die Innenseite des Tragus T, kontaktiert, ist mit A₁ bezeichnet.

Die sichelförmige Gestaltung der Stimulationselektrode 5 ist natürlich nicht zwingend. Prinzipiell kann eine asymmetrische Elektrodengeometrie im gesamten Bereich des äußeren Ohrs eingesetzt werden (also auch Cymba, Gehörgang, Tragus, etc.).

Die Referenzelektroden 6 haben eine Form, die der Region bzw. Oberfläche des Ohrs 2 angepasst ist, wo sie platziert werden sollen. Es können ovale Strukturen (wie im Beispiel der Elektrode 6") oder nierenförmige Strukturen (wie im Beispiel der Elektrode 6"') vorgesehen werden.

Die drei dargestellten Referenzelektroden 6', 6" und 6'" können alternativ oder additiv zum Einsatz kommen. Jede der Elektroden 6', 6", 6'" kontaktiert die Oberfläche 4', 4" bzw. 4'" des Ohres 2 mit einer Kontaktfläche, die mit A₂ (bzw. A₂', A₂" und A₂'" in Fig. 3) bezeichnet ist.

Wesentlich ist, dass die zweite Kontaktfläche A₂ erheblich größer ist als die erste Kontaktfläche A₁. Konkret bedeutet dies, dass die Fläche A₂ mindestens 3 Mal so groß ist wie die Fläche A₁. Wie anhand der Darstellung gemäß Fig. 3 gesehen werden kann, ist sogar ein noch viel größeres Verhältnis der Flächen vorgesehen, im Ausführungsbeispiel ein Verhältnis von mindestens 1 : 5.

Die Elektroden 5 und 6 sind in einem Abstand a im Ohr 2 angeordnet. Der minimale Abstand beträgt zumeist 5 mm. Es können aber auch Abstände bis zu 50 mm vorgesehen werden.

Angestrebt ist in jedem Falle, dass die Stimulationselektrode 5 unmittelbar an der Stelle der größten subkutanen Konzentration des aurikulären Vagusnervs angeordnet wird. Die Referenzelektrode(n) 6 wird dann im Abstand a zur Stimulationselektrode 5 in einem Nachbarareal platziert. Der Abstand a wird dabei so gewählt, dass nicht unnötig viel Körpergewebe vom Strom durchflossen wird, andererseits aber auch keine zu hohe Stromstärke benötigt wird.

Die aus Metall bestehenden Elektroden 5, 6 können in ein Elastomermaterial eingebettet sein, wofür sich ein weicher Kunststoff (z. B. Silikon oder Polyurethan) eignet, wobei eine Shore-Härte im Bereich zwischen 30 und 50 vorgesehen werden kann.

Möglich ist auch der Einsatz von elektrisch leitenden Kunststoffen statt metallischer Elektroden, was die Elektroden weicher und anpassungsfähiger macht.

Die Elektroden 5, 6 können in einer nicht dargestellte Halteanordnung integriert sein, die ins Ohr eingesetzt wird, wodurch alle vorgesehenen Elektroden 5, 6 in ihre bestimmungsgemäße Position gelangen.

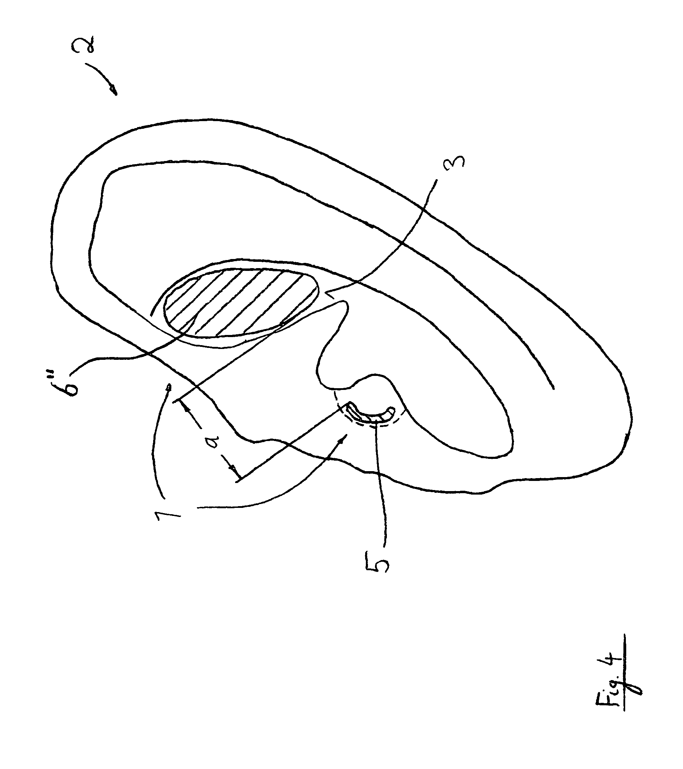

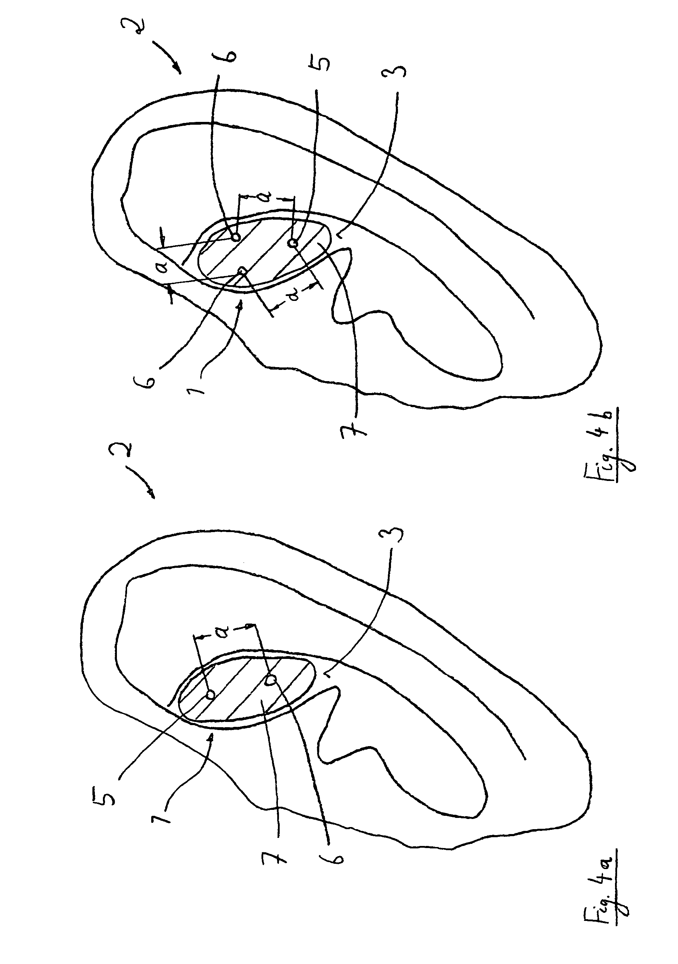

Zur Anordnung der als Anode wirkenden Referenzelektrode ist gemäß dem Ausführungsbeispiel nach Fig. 4 die Cymba conchae Cy vorgesehen, die zu mindestens 50 % ihrer Oberfläche 4" von der Referenzelektrode 6" abgedeckt wird. Bevorzugt ist die Abdeckung der Cymba conchae Cy sogar noch wesentlich größer, insbesondere mehr als 80 %. Es hat sich herausgestellt, dass eine hier aufgesetzte Elektrode 6" eine optimale Stimulationswirkung hat.

In Fig. 4a und Fig. 4b ist eine weitere Ausgestaltung der vorgeschlagenen Elektrodenanordnung illustriert. Wesentlich ist hier, dass die Elektrodenanordnung ausschließlich den Bereich der Cymba conchae Cy abdeckt bzw. kontaktiert.

Der mit Elektroden versehene Teil der Stimulationsvorrichtung weist hier einen Elektrodenträger 7 auf, der die für die Stimulation benötigen Elektroden 5, 6 trägt.

Die beiden Figuren 4a und 4b zeigen zwei Möglichkeiten der Unterbringung der Elektroden 5, 6 auf dem Elektrodenträger 7. Schematisch angedeutet sind hier lediglich punktförmige Metallelektroden, die zwischen sich einen Abstand a aufweisen und folglich nach der Beaufschlagung mit einem elektrischen Strom den zwischen sich liegenden Bereich der Cymba conchae Cy stimulieren.

Natürlich sind mannigfaltige Variationen betreffend die Anzahl und die Anordnung der Elektroden 5, 6 auf dem Elektrodenträger 7 möglich.

Wesentlich ist also bei der Lösung gemäß den Figuren 4a und 4b, dass lediglich der Bereich der Cymba conchae Cy von den Stimulationselektroden kontaktiert wird. Eine alternative Lösung, die nicht illustriert ist, stellt darauf ab, in analoger Weise ausschließlich den Bereich der Antihelix entsprechend zu stimulieren. Eine weitere alternative Ausführungsform sieht vor, dass kombiniert der Bereich der Cymba conchae und der Antihelix mit je einem Elektrodenträger 7 stimuliert wird.

Demgemäß stellen die gemäß Fig. 4a und Fig. 4b gezeigten Lösungen darauf ab, dass die Elektroden 5, 6 die Oberfläche 3 des Ohres 2 über eine Kontaktfläche kontaktieren, wobei die Elektroden 5, 6 so ausgebildet sind, dass sie mindestens 50 % der Oberfläche der Cymba conchae des Ohrs 2 abdeckt und wobei kein weiterer Bereich des Ohrs 2 mit einer Elektrode versehen ist.

Analoges gilt hinsichtlich der alternativ beschriebenen Lösung mit Blick auf die Stimulation der Antihelix.

### Bezugszeichenliste:

- 1: Elektrodenanordnung
- 2: Ohr
- 3: Oberfläche des Ohres
- 4, 4':
- 4'', 4''': Oberfläche des Ohres
- 5: Stimulationselektrode
- 6: Referenzelektrode
- 6': Referenzelektrode
- 6'': Referenzelektrode
- 6''': Referenzelektrode
- 7: Elektrodenträger

- A₁: erste Kontaktfläche
- A₂: zweite Kontaktfläche
- A₂': zweite Kontaktfläche
- A₂'': zweite Kontaktfläche
- A₂''': zweite Kontaktfläche
- a: Abstand
- AN: Antihelix
- C: Concha
- Ca: Cavum conchae
- Cy: Cymba conchae
- Cr: Crus helicis
- H: Helix
- L: Lobule
- P: Pinna
- T: Tragus

## Patentansprüche

1. Elektrodenanordnung (1) zur Anbringung am und/oder im Ohr (2) eines Menschen, wobei die Elektrodenanordnung (1) ausgebildet ist, um auf die Oberfläche (3, 4) des Ohres (2) einen transkutanen elektrischen Stimulationsreiz auszuüben, wobei die Elektrodenanordnung (1) mindestens eine Stimulationselektrode (5) und mindestens eine Referenzelektrode (6) aufweist, wobei die mindestens eine Stimulationselektrode (5) eine gebogene Struktur aufweist und die Oberfläche (3) des Ohres (2) tiber eine erste Kontaktfläche (A₁) kontaktiert, wobei die mindestens eine Referenzelektrode (6) eine ovale oder nierenförmige Struktur aufweist und die Oberfläche (4) des Ohres (2) über eine zweite Kontaktfläche (A₂) kontaktiert, wobei die zweite Kontaktfläche (A₂) mindestens 3 mal so groß ist wie die erste Kontaktfläche (A₁),
**dadurch gekennzeichnet,**
**dass** mindestens eine der Elektroden (6") die Oberfläche (3) des Ohres (2) über eine Kontaktfläche (A₂") kontaktiert, wobei die Elektrode (6") so ausgebildet ist, dass sie mindestens 50 % der Oberfläche der Cymba conchae (Cy) des Ohrs (2) abdeckt.

2. Elektrodenanordnung nach Anspruch 1, **dadurch gekennzeichnet, dass** die zweite Kontaktfläche (A₂) mindestens 5 mal so groß ist wie die erste Kontaktfläche (A₁).

3. Elektrodenanordnung nach Anspruch 1 oder 2, **dadurch gekennzeichnet, dass** die mindestens eine Stimulationselektrode (5) als Kathode und die mindestens eine Referenzelektrode (6) als Anode in dem elektrischen Stromkreis fungiert, der während der transkutanen Stimulation geschlossen ist.

4. Elektrodenanordnung nach einem der Ansprüche 1 bis 3, **dadurch gekennzeichnet, dass** die gebogene Struktur der Stimulationselektrode (5) der Umfangsform des Tragus (T) des Ohres (2) angepasst ist.

5. Elektrodenanordnung nach Anspruch 4, **dadurch gekennzeichnet, dass** die Stimulationselektrode (5) eine sichelförmige Struktur aufweist.

6. Elektrodenanordnung nach einem der Ansprüche 1 bis 5, **dadurch gekennzeichnet, dass** die ovale oder nierenförmige Struktur der Referenzelektrode (6) der Form eines weitgehend ebenen Bereichs der Oberfläche der Pinna (P) des Ohres (2) angepasst ist.

7. Elektrodenanordnung nach einem der Ansprüche 1 bis 6, **dadurch gekennzeichnet, dass** die mindestens eine Stimulationselektrode (5) und die mindestens eine Referenzelektrode (6) bei bestimmungsgemäßem Gebrauch zueinander in einem Abstand (a) angeordnet sind, der zwischen 5 mm und 50 mm beträgt.

8. Elektrodenanordnung nach einem der Ansprüche 1 bis 7, **dadurch gekennzeichnet, dass** die Stimulationselektrode (5) und die Referenzelektrode (6) aus mindestens einem metallischen Körper bestehen.

9. Elektrodenanordnung nach Anspruch 8, **dadurch gekennzeichnet, dass** der metallische Körper an oder in einem Trägerkörper angeordnet ist, der aus elastischem Material besteht.

10. Elektrodenanordnung nach Anspruch 9, **dadurch gekennzeichnet, dass** das elastische Material ein Kunststoff ist, insbesondere ein biokompatibles Elastomermaterial, besonders bevorzugt Silikon oder ein Material, das Silikon aufweist.

11. Elektrodenanordnung nach einem der Ansprüche 1 bis 7, **dadurch gekennzeichnet, dass** sie zumindest teilweise aus einem leitfähigen Kunststoff besteht.

12. Elektrodenanordnung nach einem der Ansprüche 1 bis 7, **dadurch gekennzeichnet, dass** sie aus einem Kunststoff besteht, der zumindest abschnittsweise mit einer leitfähigen Oberfläche versehen ist.

13. Elektrodenanordnung nach einem der Ansprüche 1 bis 12, **dadurch gekennzeichnet, dass** die Elektrode (6") so ausgebildet ist, dass sie mindestens 80 % der Oberfläche der Cymba conchae (Cy) des Ohrs (2) abdeckt.

14. Elektrodenanordnung nach einem der Ansprüche 1 bis 13, **dadurch gekennzeichnet, dass** eine weitere Elektrode (6') oder ein diese tragender Elektrodenträger (7) die Oberfläche (3) des Ohres (2) über eine weitere Kontaktfläche (A₂') kontaktiert, wobei die Elektrode (6') oder der Elektrodenträger (7) so ausgebildet ist, dass sie bzw. er einen Teil der Antihelix (AN) des Ohrs (2) abdeckt.

## Claims

1. Electrode arrangement (1) to be attached on and/or in the ear (2) of a human, wherein the electrode arrangement (1) is designed to exert a transcutaneous electric stimulation stimulus onto the surface (3, 4) of the ear (2), wherein the electrode arrangement (1) has at least one stimulation electrode (5) and at least one reference electrode (6), wherein the at least one stimulation electrode (5) has an arcuated structure and contacts the surface (3) of the ear (2) via a first contact surface (A₁), wherein the at least one reference electrode (6) has an oval or drused structure and contacts the surface (4) of the ear (2) via a second contact surface (A₂), wherein the second contact surface (A₂) is at least three times the size of the first contact surface (A₁),
**characterized in**
**that** at least one of the electrodes (6") contacts the surface (3) of the ear (2) via a contact surface (A₂"), wherein the electrode (6") is so designed that it covers at least 50 % of the surface of the Cymba conchae (Cy) of the ear (2).

2. Electrode arrangement according to claim 1, **characterized in that** the second contact surface (A₂) is at least five times the size of the first contact surface (A₁).

3. Electrode arrangement according to claim 1 or 2, **characterized in that** the at least one stimulation electrode (5) acts as a cathode and the at least one reference electrode (6) as an anode in the electrical circuit which is closed during the transcutaneous stimulation.

4. Electrode arrangement according to one of claims 1 to 3, **characterized in that** the arcuated structure of the stimulation electrode (5) is adapted to the outline form of the Tragus (T) of the ear (2).

5. Electrode arrangement according to claim 4, **characterized in that** the stimulation electrode (5) has a falx-shaped structure.

6. Electrode arrangement according to one of claims 1 to 5, **characterized in that** the oval or drused structure of the reference electrode (6) is adapted to the form of a substantial flat area of the surface of the Pinna (P) of the ear (2).

7. Electrode arrangement according to one of claims 1 to 6, **characterized in that** the at least one stimulation electrode (5) and the at least one reference electrode (6) are arranged with a distance (a) between 5 mm and 50 mm to another during intended use.

8. Electrode arrangement according to one of claims 1 to 7, **characterized in that** the stimulation electrode (5) and the reference electrode (6) consist of at least one metallic body.

9. Electrode arrangement according to claim 8, **characterized in that** the metallic body is arranged at or in a carrier body which consists of an elastic material.

10. Electrode arrangement according to claim 9, **characterized in that** the elastic material is a synthetic material, especially a bio compatible elastomere material, particularly preferred silicone or a material, which comprises silicone.

11. Electrode arrangement according to one of claims 1 to 7, **characterized in that** it consists at least partially of a conductive synthetic material.

12. Electrode arrangement according to one of claims 1 to 7, **characterized in that** it consists of a synthetic material which is provided at least partially with a conductive surface.

13. Electrode arrangement according to one of claims 1 to 12, **characterized in that** the electrode (6") is so designed that it covers at least 80 % of the surface of the Cymba conchae (Cy) of the ear (2).

14. Electrode arrangement according to one of claims 1 to 13, **characterized in that** a further electrode (6') or an electrode carrier (7) which bears the same contacts the surface (3) of the ear (2) via a further contact surface (A₂'), wherein the electrode (6') or the electrode carrier (7) is so designed that it covers a part of the Antihelix (AN) of the ear (2).

## Revendications

1. Arrangement d'électrodes (1) destiné à être appliqué sur et/ou dans l'oreille (2) d'une personne, l'arrangement d'électrodes (1) étant configuré pour exercer sur la surface (3, 4) de l'oreille (2) une stimulation électrique transcutanée, l'arrangement d'électrodes (1) présentant au moins une électrode de stimulation (5) et au moins une électrode de référence (6), l'au moins une électrode de stimulation (5) présentant une structure courbée et venant en contact avec la surface (3) de l'oreille (2) par le biais d'une première surface de contact (A₁), l'au moins une électrode de référence (6) présentant une structure ovale ou réniforme et venant en contact avec la surface (4) de l'oreille (2) par le biais d'une deuxième surface de contact (A₂), la deuxième surface de contact (A₂) étant au moins 3 fois plus grande que la première surface de contact (A₁),
**caractérisé en ce**
**qu'**au moins l'une des électrodes (6") vient en contact avec la surface (3) de l'oreille (2) par le biais d'une surface de contact (A₂"), l'électrode (6") étant configurée de telle sorte qu'elle recouvre au moins 50 % de la surface de la cymba conchae (Cy) de l'oreille (2).

2. Arrangement d'électrodes selon la revendication 1, **caractérisé en ce que** la deuxième surface de contact (A₂) est au moins 5 fois plus grande que la première surface de contact (A₁).

3. Arrangement d'électrodes selon la revendication 1 ou 2, **caractérisé en ce que** l'au moins une électrode de stimulation (5) fait office de cathode et l'au moins une électrode de référence (6) d'anode dans le circuit électrique qui est fermé pendant la stimulation transcutanée.

4. Arrangement d'électrodes selon l'une des revendications 1 à 3, **caractérisé en ce que** la structure courbée de l'électrode de stimulation (5) est adaptée à la forme du contour du tragus (T) de l'oreille (2).

5. Arrangement d'électrodes selon la revendication 4, **caractérisé en ce que** l'électrode de stimulation (5) présente une structure en forme de croissant.

6. Arrangement d'électrodes selon l'une des revendications 1 à 5, **caractérisé en ce que** la structure ovale ou réniforme de l'électrode de référence (6) est adaptée à la forme d'une zone largement plane de la surface du pavillon (P) de l'oreille (2).

7. Arrangement d'électrodes selon l'une des revendications 1 à 6, **caractérisé en ce que** l'au moins une électrode de stimulation (5) et l'au moins une électrode de référence (6), lors d'une utilisation conforme à l'usage prévu, sont disposées avec un écart (a) entre elles qui est compris entre 5 mm et 50 mm.

8. Arrangement d'électrodes selon l'une des revendications 1 à 7, **caractérisé en ce que** l'électrode de stimulation (5) et l'électrode de référence (6) se composent d'au moins un corps métallique.

9. Arrangement d'électrodes selon la revendication 8, **caractérisé en ce que** le corps métallique est disposé sur ou dans un corps porteur qui se compose d'un matériau élastique.

10. Arrangement d'électrodes selon la revendication 9, **caractérisé en ce que** le matériau élastique est une matière plastique, notamment un matériau élastomère biocompatible, notamment de préférence du silicone ou un matériau qui présente du silicone.

11. Arrangement d'électrodes selon l'une des revendications 1 à 7, **caractérisé en ce qu'**il se compose au moins partiellement d'une matière plastique conductrice.

12. Arrangement d'électrodes selon l'une des revendications 1 à 7, **caractérisé en ce qu'**il se compose d'une matière plastique qui est munie au moins sur certaines portions d'une surface conductrice.

13. Arrangement d'électrodes selon l'une des revendications 1 à 12, **caractérisé en ce que** l'électrode (6") est configurée de telle sorte qu'elle recouvre au moins 80 % de la surface de la cymba conchae (Cy) de l'oreille (2).

14. Arrangement d'électrodes selon l'une des revendications 1 à 13, **caractérisé en ce qu'**une électrode supplémentaire (6') ou un porte-électrode (7) supportant celle-ci vient en contact avec la surface (3) de l'oreille (2) par le biais d'une surface de contact supplémentaire (A₂'), l'électrode (6') ou le porte-électrode (7) étant configuré(e) de telle sorte qu'elle ou il recouvre une partie de l'anti-hélix (AN) de l'oreille (2).
